# EUROPEAN PATENT APPLICATION

(11) **EP 4 495 287 A1**
(43) Date of publication of application: **22.01.2025**
(21) Application number: 23770971.2
(22) Date of filing: 10.02.2023
(51) Int. Cl.: C23C 16/455, C23C 16/04, C23C 16/40, C23C 16/34, H01L 21/768

(54) **SHIELDING COMPOUND, THIN FILM FORMING METHOD USING SAME, AND SEMICONDUCTOR SUBSTRATE AND SEMICONDUCTOR DEVICE MANUFACTURED THEREFROM**

(30) Priority: 16.03.2022 KR 20220032781; 27.06.2022 KR 20220078082
(71) Applicant: Soulbrain Co., Ltd., Seongnam-si, Gyeonggi-do 13486 (KR)
(72) Inventor: LEE, Seung Hyun, Seongnam-si, Gyeonggi-do 13486 (KR); JUNG, Jae Sun, Seongnam-si, Gyeonggi-do 13486 (KR); YEON, Chang Bong, Seongnam-si, Gyeonggi-do 13486 (KR); TAN, Kok Chew, Seongnam-si, Gyeonggi-do 13486 (KR); CHO, Deok Hyun, Seongnam-si, Gyeonggi-do 13486 (KR); NAM, Ji Hyun, Seongnam-si, Gyeonggi-do 13486 (KR)
(74) Representative: Dehns
(86) International application number: PCT/KR2023/001950
(87) International publication number: WO 2023/177091

(57) **Abstract**

The present invention relates to a shielding compound, a method of forming a thin film using the shielding compound, a semiconductor substrate fabricated using the method, and a semiconductor device including the semiconductor substrate. According to the present invention, by providing a compound having a predetermined structure as a shielding agent, by forming a deposition layer with a uniform thickness as a shielded area on a substrate due to the difference in adsorption distribution of the shielding agent, the deposition rate of the thin film may be reduced, and the thin film growth rate may be appropriately reduced. In addition, even when forming a thin film on a substrate with a complex structure, step coverage and the thickness uniformity of a thin film may be greatly improved, and impurities may be reduced.

## Description

### [Technical Field]

The present invention relates to a shielding compound, a method of forming a thin film using the shielding compound, a semiconductor substrate fabricated using the method, and a semiconductor device including the semiconductor substrate. More particularly, according to the present invention, by providing a compound having a predetermined structure as a shielding agent, by forming a deposition layer with a uniform thickness as a shielded area on a substrate due to the difference in adsorption distribution of the shielding agent, the deposition rate of the thin film may be reduced, and the thin film growth rate may be appropriately reduced. In addition, even when forming a thin film on a substrate with a complex structure, step coverage and the thickness uniformity of a thin film may be improved, and impurities may be greatly reduced.

### [Background Art]

As the integration of memory and non-memory semiconductor devices increases, the microstructure of a substrate is becoming increasingly complex.

For example, the ratio of the width to depth of the microstructure (hereinafter referred to as the 'aspect ratio') is increasing to 20:1 or more, and even to 100:1 or more. As the aspect ratio increases, it becomes difficult to form a deposition layer with a uniform thickness along the plane of the complex microstructure.

Accordingly, step coverage, which defines the thickness ratio of deposition layers formed at the top and bottom in the depth direction of the microstructure, remains at the level of 90 %. Accordingly, the expression of the electrical characteristics of a device becomes increasingly difficult. Since a step coverage of 100 % means that deposition layers formed on the upper and lower parts of the microstructure have the same thickness, it is necessary to develop a technology that can achieve step coverage close to 100 %.

Thin films for semiconductors are made of nitride films, oxide films, metal films, etc. Examples of nitride films include silicon nitride (SiN), titanium nitride (TiN), and tantalum nitride (TaN); examples of oxide films include silicon oxide (SiO₂), hafnium oxide (HfO₂), and zirconium oxide (ZrO₂); and examples of metal films include molybdenum (Mo) and tungsten (W).

The thin film is generally used as a diffusion barrier between the silicon layer of a doped semiconductor and aluminum (Al), copper (Cu), etc., which are used as interlayer wiring materials. However, when depositing a tungsten (W) thin film on a substrate, the thin film is used as an adhesion layer.

In addition, as described above, to provide excellent and uniform physical properties to a thin film deposited on a substrate, it is essential that the thin film has high step coverage. Accordingly, the atomic layer deposition (ALD) process, which uses surface reactions, is used rather than the chemical vapor deposition (CVD) process, which mainly uses gaseous reactions. However, there are still problems in implementing 100 % step coverage.

When increasing deposition temperature to achieve 100 % step coverage, it is difficult to achieve step coverage. First, in a deposition process using a precursor and a reactant, an increase in the deposition temperature leads to a steep increase in the thin film growth rate (GPC). In addition, even when the ALD process is performed at 300 °C to alleviate the increase in GPC due to the increase in deposition temperature, the deposition temperature increases during the process. Accordingly, the problem still remains.

Accordingly, methods of reducing the growth rate of thin films have been proposed. However, when deposition temperature is reduced to reduce the growth rate of a thin film, a problem occurs in which the film quality significantly deteriorates due to an increase in the residual amount of impurities, such as carbon or chlorine, within the thin film (see paper, J. Vac. Sci. Technol. A, 35(2017) 01B130).

According to the literature, process by-products such as chloride remain in the manufactured thin film, causing corrosion of metals such as aluminum, and causing deterioration of film quality due to the generation of nonvolatile by-products.

Therefore, it is necessary to develop a method of forming a thin film that allows the formation of a thin film with a complex structure, reduces the residual amount of impurities, and greatly improves step coverage and the thickness uniformity of a thin film; a semiconductor substrate fabricated using the method; and a semiconductor device including the semiconductor substrate.

### [Disclosure]

### [Technical Problem]

Therefore, the present invention has been made in view of the above problems, and it is one object of the present invention to provide a shielding compound, a method of forming a thin film using the shielding compound, a semiconductor substrate fabricated using the method, and a semiconductor device including the semiconductor substrate. According to the present invention, by forming a deposition layer with a uniform thickness on a substrate by the difference in adsorption distribution of a predetermined shielding agent as a shielded area for thin films, the deposition rate of the thin film may be reduced, and the thin film growth rate may be appropriately reduced. In addition, even when forming a thin film on a substrate with a complex structure, step coverage and the thickness uniformity of a thin film may be greatly improved.

It is another object of the present invention to improve the density, electrical properties, and dielectric properties of a thin film by improving the crystallinity and oxidation fraction of the thin film.

The above and other objects can be accomplished by the present invention described below.

### [Technical Solution]

In accordance with one aspect of the present invention, provided is a thin film shielding compound having two or more types of nitrogen (N), oxygen (O), phosphorus (P), and sulfur (S) and including a linear or cyclic saturated or unsaturated hydrocarbon having 3 to 15 carbon atoms.

The thin film shielding compound may have a structure containing nitrogen (N), oxygen (O), phosphorus (P), or sulfur (S) at each terminal of a central carbon atom connected by a double bond to oxygen.

The thin film shielding compound may have a structure containing nitrogen (N), oxygen (O), phosphorus (P), or sulfur (S) at one end of a central carbon atom connected by a double bond to oxygen and carbon (C) at the other end.

The thin film shielding compound may include one or more selected from a compound represented by Chemical Formula 1 below and a compound represented by Chemical Formula 2 below. 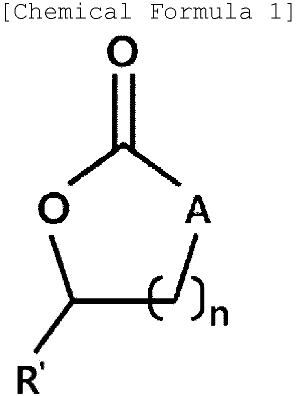 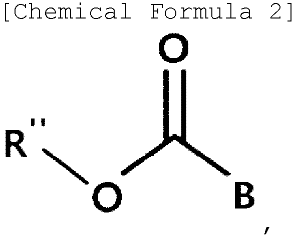
wherein A is oxygen (O), sulfur (S), phosphorus (P), nitrogen (N), -CH, or -CH₂;
B is -OH, -OCH₃, -OCH₂CH₃, -CH₂CH₃, -SH, -SCH₃, or - SCH₂CH₃;
R' and R'' are independently hydrogen, an alkyl group having 1 to 5 carbon atoms, an alkene group having 1 to 5 carbon atoms, or an alkoxy group having 1 to 5 carbon atoms; and
n is an integer from 0 to 3.

The shielding compound may have a deposition rate reduction rate of 30 % or more as calculated by Equation 1 below. Deposition rate reduction rate = [{(DRn)-(DRw)}/(DRn)] × 100

In Equation 1, DRn is the depth rate measured on the manufactured thin film without adding the shielding compound, and DRw is the depth rate measured on the manufactured thin film with the addition of the shielding compound. Here, the depth rate is measured at room temperature and pressure using an ellipsometer for a thin film with a thickness of 3 to 30 nm, and the unit is Å/cycle.

The shielding compound may have a refractive index of 1.39 or less, 1.41 to 1.42, 1.43 to 1.44, or 1.45 to 1.46.

The shielding compound may include one or more selected from compounds represented by Chemical Formulas 1-1 to 1-4 and Chemical Formulas 2-1 to 2-4 below.

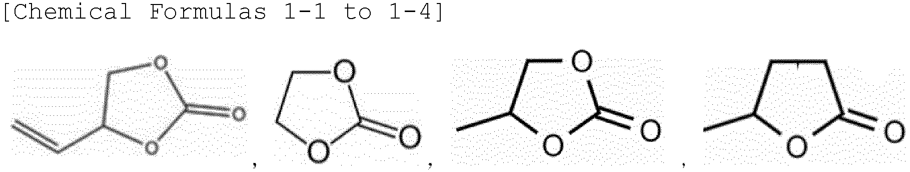

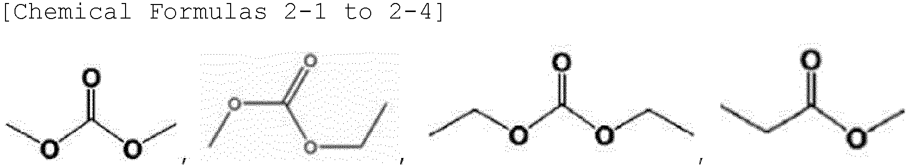

The shielding compound may provide a shielded area for an oxide film, a nitride film, a metal film, or a selective thin film thereof.

The shielded area may be formed on the entire or part of a substrate on which the oxide film, the nitride film, the metal film, or the selective thin film thereof is formed.

Based on 100 % of a total area of the substrate, the shielded area may occupy 10 to 95 % of the entire substrate or a portion of the substrate, and an unshielded area may occupy the remainder.

When a total area of the substrate is 100 %, a first shielded area may occupy 10 to 95 % of a total area of the entire substrate or a portion of the substrate, a second shielded area may occupy 10 to 95 % of the remaining area, and an unshielded area may occupy the remaining area.

The thin film may be a laminated film of one or more selected from the group consisting of Al, Si, Ti, V, Co, Ni, Cu, Zn, Ga, Ge, Se, Zr, Nb, Mo, Ru, Rh, In, Sn, Sb, Te, Hf, Ta, W, Re, Os, Ir, La, Ce, and Nd.

The thin film may be used as a diffusion barrier film, an etching stop film, an electrode film, a dielectric film, a gate insulating film, a block oxide film, or a charge trap.

In accordance with another aspect of the present invention, provided is a method of forming a thin film, the method including injecting a shielding compound having a structure represented by Chemical Formula 1 or 2 below into a chamber to shield a surface of a loaded substrate. 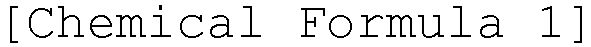 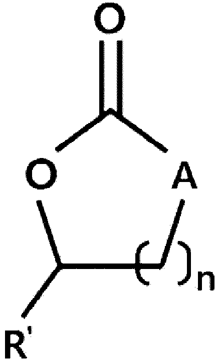 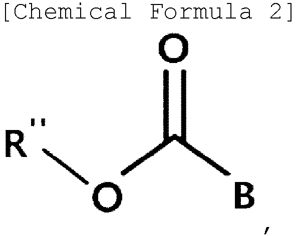
wherein A is oxygen (O), sulfur (S), phosphorus (P), nitrogen (N), -CH, or -CH₂;
B is -OH, -OCH₃, -OCH₂CH₃, -CH₂CH₃, -SH, -SCH₃, or - SCH₂CH₃;
R' and R'' are independently hydrogen, an alkyl group having 1 to 5 carbon atoms, an alkene group having 1 to 5 carbon atoms, or an alkoxy group having 1 to 5 carbon atoms; and n is an integer from 0 to 3.

In accordance with still another aspect of the present invention, provided is a method of forming a thin film, the method including:
i) vaporizing the above-described shielding compound to form a shielded area on a surface of a substrate loaded in a chamber;
ii) performing 1st purging inside the chamber with a purge gas;
iii) vaporizing a precursor compound and adsorbing the precursor compound onto an area outside the shielded area;
iv) performing 2nd purging inside the chamber with a purge gas;
v) supplying a reaction gas inside the chamber; and
vi) performing 3rd purging inside the chamber with a purge gas.

In accordance with still another aspect of the present invention, provided is a method of forming a thin film, the method including:
i) vaporizing a precursor compound and adsorbing the precursor compound onto a surface of a substrate loaded into a chamber;
ii) performing 1st purging inside the chamber with a purge gas;
iii) vaporizing the shielding compound to shield the surface of the substrate loaded in the chamber;
iv) performing 2nd purging inside the chamber with a purge gas;
v) supplying a reaction gas inside the chamber; and
vi) performing 3rd purging inside the chamber with a purge gas.

The precursor compound may be a molecule composed of one or more selected from the group consisting of Al, Si, Ti, V, Co, Ni, Cu, Zn, Ga, Ge, Se, Zr, Nb, Mo, Ru, Rh, In, Sn, Sb, Te, Hf, Ta, W, Re, Os, Ir, La, Ce, and Nd, and may be a precursor having a vapor pressure of greater than 0.01 mTorr and 100 Torr or less at 25 °C.

The chamber may be an ALD chamber, a CVD chamber, a PEALD chamber, or a PECVD chamber.

The shielding compound or the precursor compound may be vaporized, injected, and then subjected to plasma post-treatment.

In steps ii) and iv), an amount of the purge gas injected into the chamber at each step may be 10 to 100,000 times a volume of the introduced shielding compound.

The reaction gas may be a nitriding agent, an oxidizing agent, or a reducing agent, and the reaction gas, the shielding compound, and the precursor compound may be transferred into the chamber by a VFC, DLI, or LDS method.

The thin film may be a silicon nitride film, a silicon oxide film, a titanium nitride film, a titanium oxide film, a tungsten nitride film, a molybdenum nitride film, a hafnium oxide film, a zirconium oxide film, a tungsten oxide film, or an aluminum oxide film.

The substrate loaded into the chamber may be heated to 100 to 800 °C, and the ratio of amount (mg/cycle) of the shielding compound and the precursor compound fed into the chamber may be 1 : 1 to 1 : 20.

In accordance with still another aspect of the present invention, provided is a semiconductor substrate fabricated using the above-described method of forming a thin film.

The thin film may have a multilayer structure of 2 or 3 layers.

In accordance with yet another aspect of the present invention, provided is a semiconductor device including the above-described semiconductor substrate.

The semiconductor substrate may be low resistive metal gate interconnects, a high aspect ratio 3D metal-insulator-metal capacitor, a DRAM trench capacitor, 3D Gate-All-Around (GAA), or a 3D NAND flash memory.

### [Advantageous Effects]

According to the present invention, the present invention has an effect of providing a shielding compound that improves step coverage even when forming a thin film on a substrate with a complex structure by forming a shielded area for thin films on the substrate to reduce a thin film deposition rate and reduce a thin film growth rate appropriately.

In addition, by more effectively reducing process by-products during thin film formation, corrosion and deterioration can be prevented, the crystallinity of a thin film can be improved, and the electrical properties of the thin film can be improved.

In addition, when forming a thin film, process by-products can be reduced, and step coverage and thin film density can be improved. In addition, the present invention has an effect of providing a method of forming a thin film using the shielding compound, a semiconductor substrate fabricated using the method, and a semiconductor device including the semiconductor substrate.

### [Description of Drawings]

FIG. 1 is an SIMS analysis graph for an HfO₂ thin film manufactured in Example 4.
FIG. 2 is an SIMS analysis graph for an HfO₂ thin film manufactured in Comparative Example 4.
FIG. 3 is a graph analyzing the hafnium (Hf) to oxygen (O) ratio of a thin film manufactured in Comparative Example 3.
FIG. 4 is a graph analyzing the hafnium (Hf) to oxygen (O) ratio of a thin film manufactured in Comparative Example 8.
FIG. 5 is a TEM measurement diagram of a specimen cut horizontally at a position 100 nm downward from the upper portion (left diagram) and at a position 100 nm upward from the lower portion (right diagram) of a thin film deposited under the conditions of Example 4 on a substrate having a complex structure with an aspect ratio of 22:1.
FIG. 6 is a TEM measurement diagram of a specimen cut horizontally at a position 100 nm downward from the upper portion (left diagram) and at a position 100 nm upward from the lower portion (right diagram) of a thin film deposited under the conditions of Example 5 on a substrate having a complex structure with an aspect ratio of 22:1.
FIG. 7 is a diagram showing the XRD crystal patterns of Comparative Example 4 (black) and Example 1 (blue).

### [Best Mode]

Hereinafter, a shielding compound of the present invention, a method of forming a thin film using the shielding compound, a semiconductor substrate including the thin film, and a semiconductor device including the semiconductor substrate will be described in detail.

In the present disclosure, unless otherwise specified, the term "shielding" means reducing, inhibiting, or blocking adsorption of precursor compounds for forming a thin film onto a substrate, and also reducing, inhibiting, or blocking adsorption of process by-products onto the substrate.

The present inventors confirmed that, by providing a compound having a predetermined structure as a shielding agent that shields a precursor compound for forming a thin film on the surface of a substrate loaded inside a chamber, by forming a deposition layer with a uniform thickness due to the difference in adsorption distribution of the shielding agent as a shielded area that does not remain in the thin film, as a relatively coarse thin film was formed, the growth rate of a thin film formed at the same time was greatly reduced, so that even when applied to a substrate having a complex structure, the uniformity of the thin film was secured, and the step coverage was greatly improved. In particular, thin-thickness deposition was possible, and the remaining O, Si, metal, and metal oxides as process by-products and carbon residues, which were difficult to reduce in the past, were reduced. Based on these results, the present inventors conducted further studies on a shielding compound for providing a shielded area to complete the present invention.

The shielding compound of the present invention provides a shielding compound for thin films.

For example, the thin film may be provided with one or more precursors selected from the group consisting of Al, Si, Ti, V, Co, Ni, Cu, Zn, Ga, Ge, Se, Zr, Nb, Mo, Ru, Rh, In, Sn, Sb, Te, Hf, Ta, W, Re, Os, Ir, La, Ce, and Nd, and may provide a shielded area for an oxide film, a nitride film, a metal film, or a selective thin film thereof. In this case, the effects desired in the present invention may be sufficiently achieved.

As a specific example, the thin film may have a film composition of a silicon nitride film, a silicon oxide film, a titanium nitride film, a titanium oxide film, a tungsten nitride film, a molybdenum nitride film, a hafnium oxide film, a zirconium oxide film, a tungsten oxide film, or an aluminum oxide film.

The thin film may contain the aforementioned film composition alone or in a selective area, but is not limited thereto, and also includes SiH and SiOH.

In addition to a commonly used diffusion barrier film, the thin film may be used in semiconductor devices as an etching stop film, an electrode film, a dielectric film, a gate insulating film, a block oxide film, or a charge trap.

The shielding compound has two or more types of nitrogen (N), oxygen (O), phosphorus (P), and sulfur (S), and includes a linear or cyclic saturated or unsaturated hydrocarbon having 3 to 15 carbon atoms. In this case, by forming a shielded area that does not remain in a thin film during thin film formation, a relatively coarse thin film may be formed and side reactions may be suppressed. In addition, by controlling the thin film growth rate, process by-products within the thin film may be reduced, thereby reducing corrosion and deterioration. In addition, the crystallinity of the thin film may be improved, and a stoichiometric oxidation state may be reached when a metal oxide film is formed. In addition, even when a thin film is formed on a substrate having a complex structure, step coverage and the thickness uniformity of a thin film may be greatly improved.

As a specific example, the shielding compound has a structure containing nitrogen (N), oxygen (O), phosphorus (P), or sulfur (S) at each terminal of a central carbon atom connected by a double bond to oxygen. Thus, the effect of reducing process by-products and the effect of improving thin film density may be increased, and step coverage and the electrical properties of the thin film may be excellent.

Here, unless otherwise specified, the structure containing nitrogen (N), oxygen (O), phosphorus (P), or sulfur (S) at each terminal of a central carbon atom connected by a double bond to oxygen refers to

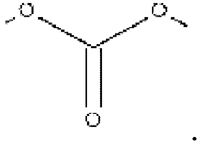

As a specific example, the shielding compound has a structure containing nitrogen (N), oxygen (O), phosphorus (P), or sulfur (S) at one end of a central carbon atom connected by a double bond to oxygen and carbon (C) at the other end. Thus, the effect of reducing process by-products and the effect of improving thin film density may be increased, and step coverage and the electrical properties of the thin film may be excellent.

Here, unless otherwise specified, the structure containing nitrogen (N), oxygen (O), phosphorus (P), or sulfur (S) at one end of a central carbon atom connected by a double bond to oxygen and carbon (C) at the other end refers to

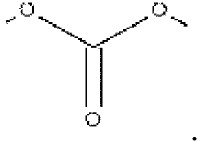

As a specific example, the shielding compound may include one or more selected from a compound represented by Chemical Formula 1 below and a compound represented by Chemical Formula 2 below. In this case, by forming a shielded area that does not remain in a thin film during thin film formation, a relatively coarse thin film may be formed and side reactions may be suppressed. In addition, by controlling the thin film growth rate, process by-products within the thin film may be reduced, thereby reducing corrosion and deterioration. In addition, the crystallinity of a thin film may be improved. In addition, even when a thin film is formed on a substrate having a complex structure, step coverage and the thickness uniformity of a thin film may be greatly improved. 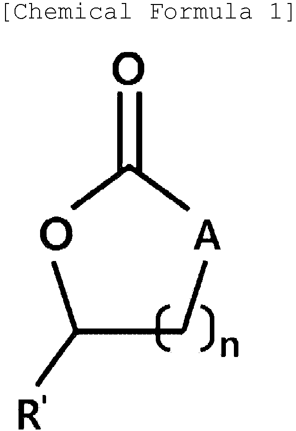 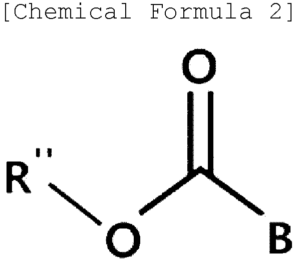
Here, A is oxygen (O), sulfur (S), phosphorus (P), nitrogen (N), -CH, or -CH₂;
B is -OH, -OCH₃, -OCH₂CH₃, -CH₂CH₃, -SH, -SCH₃, or - SCH₂CH₃;
R' and R'' are independently hydrogen, an alkyl group having 1 to 5 carbon atoms, an alkene group having 1 to 5 carbon atoms, or an alkoxy group having 1 to 5 carbon atoms; and
n is an integer from 0 to 3.

In Chemical Formula 1, A is oxygen (O) or sulfur (S), preferably oxygen (O). In this case, the effect of reducing process by-products and the effect of improving thin film density may be increased, and step coverage and the electrical properties, insulating properties, and dielectric properties of the thin film may be excellent.

R' is an alkyl group having 1 to 3 carbon atoms, preferably an alkyl group having 1 to 2 carbon atoms. In this case, the effect of reducing process by-products and the effect of improving thin film density may be increased, and step coverage and the electrical properties, insulating properties, and dielectric properties of the thin film may be excellent.

n is an integer from 1 to 2, preferably an integer of 1.

In Chemical Formula 2, B is -OH, -OCH₃, -OCH₂CH₃, - CH₂CH₃, -SH, -SCH₃, or -SCH₂CH₃. In this case, the effect of reducing process by-products may be increased, step coverage may be excellent, and the effect of improving the density and electrical properties of a thin film may be excellent.

R" is an alkyl group having 1 to 3 carbon atoms, preferably an alkyl group having 1 to 2 carbon atoms. In this case, the effect of reducing process by-products may be increased, step coverage may be excellent, and the effect of improving the density and electrical properties of a thin film may be excellent.

When the compounds represented by Chemical Formulas 1 and 2 are formed into a saturated structure, the deposition process efficiency may be improved.

The compounds represented by Chemical Formulas 1 and 2 may exclude structures containing halogen.

The shielding compound, preferably the compounds represented by Chemical Formulas 1 and 2 may have a deposition rate reduction rate of 30 % or more, as a specific example, 35 % or more, preferably 38 % or more as calculated by Equation 1 below. In this case, by forming a deposition layer with a uniform thickness due to the difference in the adsorption distribution of the shielding agent having the aforementioned structure as a shielded area that does not remain in a thin film, as a relatively coarse thin film is formed, the growth rate of a thin film formed at the same time is greatly reduced, so that even when applied to a substrate having a complex structure, the uniformity of the thin film may be secured, and the step coverage may be greatly improved. In particular, deposition in a thin thickness is possible, and the remaining amounts of O, Si, metals, and metal oxides remaining as process by-products may be improved. In addition, even the remaining amount of carbon, which was difficult to reduce in the past, may be improved. Deposition rate reduction rate = [{(DRn)-(DRw)}/(DRn)] × 100

In Equation 1, DRn is the depth rate measured on the manufactured thin film without adding the shielding compound, and DRw is the depth rate measured on the manufactured thin film with the addition of the shielding compound. Here, the depth rate is measured at room temperature and pressure using an ellipsometer for a thin film with a thickness of 3 to 30 nm, and the unit is Å/cycle.

In Equation 1, when the shielding compound is used and when the shielding compound is not used, for each case, the thin film growth rate per cycle means the thin film deposition thickness (Å/cycle) per cycle, i.e., the deposition rate. For example, the deposition rate may be obtained as an average deposition rate calculated by measuring the final thickness of a thin film with a thickness of 3 to 30 nm under room temperature and pressure conditions using an ellipsometer, and then dividing the final thickness by the total number of cycles.

In Equation 1, "when the shielding compound is not used" means that a thin film is manufactured by adsorbing only a precursor compound on a substrate in a thin film deposition process. As a specific example, in the thin film forming method, the above case refers to a case where a thin film is formed by omitting a step of adsorbing a shielding compound and a step of purging an unadsorbed shielding compound.

For example, when the shielding compound is a compound represented by Chemical Formula 1, the shielding compound may be a compound having a refractive index of 1.4 to 1.42 or 1.43 to 1.5, as a specific example, 1.41 to 1.417 or 1.43 to 1.47, preferably 1.413 to 1.417 or 1.450 to 1.452.

For example, when the shielding compound is a compound represented by Chemical Formula 2, the shielding compound may be a compound having a refractive index of 1.39 or less, as a specific example, 1.3 to 1.39, preferably 1.35 to 1.385.

In this case, by forming, as a shielded area that does not remain in the thin film, a deposition layer of uniform thickness due to the difference in adsorption distribution of the shielding agent having the aforementioned structure on the substrate, the deposition rate of the thin film may be reduced, and the thin film growth rate may be appropriately reduced. Thus, even when a thin film is formed on a substrate having a complex structure, step coverage and the thickness uniformity of a thin film may be greatly improved. In addition to a thin film precursor, the surface of the substrate may be effectively protected by preventing the adsorption of process by-products, and process by-products may be effectively removed.

In particular, as a relatively coarse thin film is formed, the growth rate of a thin film formed at the same time is greatly reduced, so that even when applied to a substrate having a complex structure, the uniformity of the thin film may be secured, and the step coverage may be greatly improved. In particular, deposition in a thin thickness is possible, and the remaining amounts of O, Si, metals, and metal oxides remaining as process by-products may be improved. In addition, even the remaining amount of carbon, which was difficult to reduce in the past, may be improved.

The compound represented by Chemical Formula 1 may include one or more selected from compounds represented by Chemical Formulas 1-1 to 1-4 below. In this case, by providing a thin film-shielded area, the growth rate of a thin film may be effectively controlled, and process by-products may be effectively removed. In addition, step coverage and film quality may be greatly improved.

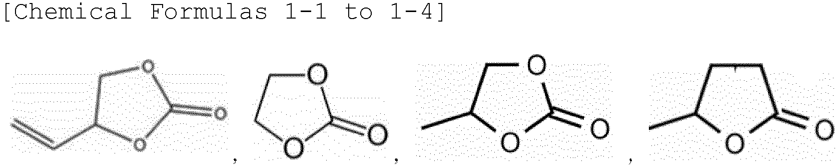

The compound represented by Chemical Formula 2 may include one or more selected from compounds represented by Chemical Formulas 2-1 to 2-4 below. In this case, by providing a thin film-shielded area, the growth rate of a thin film may be effectively controlled, and process by-products may be effectively removed. In addition, step coverage and film quality may be greatly improved.

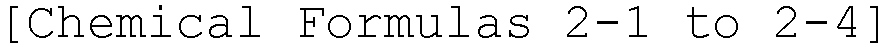

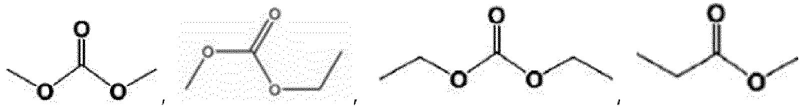

The shielding compound may provide a shielded area for thin films.

For example, the shielded area for thin films may be formed on the entire substrate or a portion of the substrate on which the thin film is formed.

In addition, when a total area of the substrate is 100 %, the shielded area for thin films may occupy 10 to 95 %, as a specific example, 15 to 90 %, preferably 20 to 85 %, more preferably 30 to 80 %, still more preferably 40 to 75 %, still more preferably 40 to 70 % of a total area of the entire substrate or a portion of the substrate, and the unshielded area may occupy the remining area.

In addition, when a total area of the substrate is 100 %, a first shielded area may occupy 10 to 95 %, as a specific example, 15 to 90 %, preferably 20 to 85 %, more preferably 30 to 80 %, still more preferably 40 to 75 %, still more preferably 40 to 70 % of a total area of the entire substrate or a portion of the substrate, a second shielded area may occupy 10 to 95 %, as a specific example, 15 to 90 %, preferably 20 to 85 %, more preferably 30 to 80 %, still more preferably 40 to 75 %, still more preferably 40 to 70 % of the remaining area, and an unshielded area may occupy the remaining area.

The shielded area for thin films does not remain on the thin film.

At this time, unless otherwise specified, non-residue refers to a case where the content of C element is less than 0.1 atom %, the content of Si element is less than 0.1 atom %, the content of N element is less than 0.1 atom %, and the content of halogen element is less than 0.1 atom % when analyzed by XPS. More preferably, in the secondary-ion mass spectrometry (SIMS) measurement method or X-ray photoelectron spectroscopy (XPS) measurement method, in which measurements are performed in the depth direction of a substrate, considering the increase/decrease rate of C, N, Si, and halogen impurities before and after using the shielding agent under the same deposition conditions, it is desirable that the increase/decrease rate of the signal sensitivity (intensity) of each element type does not exceed 5 %.

For example, the thin film may include a halogen compound in an amount of 100 ppm or less.

The thin film may be used as an etching stop film, an electrode film, a dielectric film, a gate insulating film, a block oxide film, or a charge trap, without being limited thereto.

The shielding compound may be preferably a compound having a purity of 99.9 % or more, 99.95 % or more, or 99.99 % or more. For reference, when a compound having a purity of less than 99 % is used, impurities may remain in a thin film or cause side reactions with precursors or reactants. Accordingly, it is desirable to use a material having a purity of 99 % or more.

The shielding compound is preferably used in the atomic layer deposition (ALD) process. In this case, the surface of a substrate may be effectively protected and process by-products may be effectively removed without interfering with the adsorption of a precursor compound.

The shielding compound may be preferably liquid at room temperature (22 °C), and may have a density of 0.8 to 2.5 g/cm³ or 0.8 to 1.5 g/cm³ and a vapor pressure (20 °C) of 0.1 to 300 mmHg or 1 to 300 mmHg. Within this range, a shielded area may be effectively formed, and the step coverage and the thickness uniformity and film quality of a thin film may be greatly improved.

More preferably, the shielding compound may have a density of 0.75 to 2.0 g/cm³ or 0.8 to 1.3 g/cm³ and a vapor pressure (20 °C) of 1 to 260 mmHg. Within this range, a shielded area may be effectively formed, and the step coverage and the thickness uniformity and film quality of a thin film may be greatly improved.

The method of forming a thin film according to the present invention includes a step of injecting a shielding compound having a structure represented by Chemical Formula 1 or 2 below into a chamber to shield a surface of a loaded substrate. In this case, by forming a shielded area for thin films on the substrate, the deposition rate of the thin film may be reduced, and the thin film growth rate may be appropriately reduced. In addition, even when forming a thin film on a substrate with a complex structure, step coverage and the thickness uniformity of a thin film may be greatly improved. 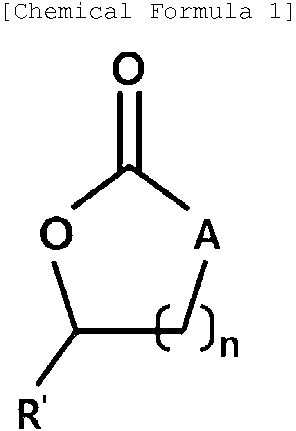 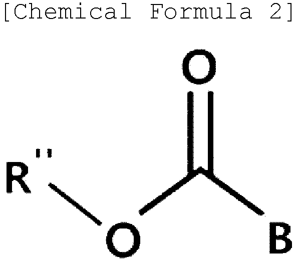
Here, A is oxygen (O), sulfur (S), phosphorus (P), nitrogen (N), -CH, or -CH₂;
B is -OH, -OCH₃, -OCH₂CH₃, -CH₂CH₃, -SH, -SCH₃, or - SCH₂CH₃;
R' and R'' are independently hydrogen, an alkyl group having 1 to 5 carbon atoms, an alkene group having 1 to 5 carbon atoms, or an alkoxy group having 1 to 5 carbon atoms; and n is an integer from 0 to 3.

In the step of shielding using the shielding compound on the surface of the substrate, the feeding time (sec) of the shielding compound on the surface of the substrate may be preferably 0.01 to 5 seconds, more preferably 0.02 to 3 seconds, still more preferably 0.04 to 2 seconds, still more preferably 0.05 to 1 seconds per cycle. Within this range, thin film growth rate may be reduced, and step coverage and economics may be excellent.

In the present disclosure, the feeding time of the shielding compound is based on a flow rate of 0.1 to 50 mg/cycle a chamber volume of 15 to 20 L, more specifically based on a flow rate of 0.8 to 20 mg/cycle at a chamber volume of 18 L.

As a preferred example, the method of forming a thin film may include step i) of vaporizing the shielding compound to shield the surface of a substrate loaded in the chamber; step ii) of performing 1st purging inside the chamber with a purge gas; step iii) of vaporizing a precursor compound and adsorbing the precursor compound onto a surface of the substrate loaded into the chamber; step iv) of performing 2nd purging inside the chamber with a purge gas; step v) of supplying a reaction gas inside the chamber; and step vi) of performing 3rd purging inside the chamber with a purge gas. At this time, steps i) to vi) may be repeated as a unit cycle until a thin film of the desired thickness is obtained. In this way, in one cycle, when the shielding compound of the present invention is injected before the precursor compound and is absorbed into the substrate, even when deposition is performed at high temperatures, the thin film growth rate may be appropriately reduced, process by-products may be effectively removed, the resistivity of the thin film may be reduced, and the step coverage may be significantly improved.

As another preferred example, the method of forming a thin film may include step i) of vaporizing a precursor compound and adsorbing the precursor compound onto a surface of a substrate loaded into a chamber; step ii) of performing 1st purging inside the chamber with a purge gas; step iii) of vaporizing the shielding compound and adsorbing the shielding compound onto the surface of the substrate loaded in the chamber; step iv) of performing 2nd purging inside the chamber with a purge gas; step v) of supplying a reaction gas inside the chamber; and step vi) of performing 3rd purging inside the chamber with a purge gas. At this time, steps i) to vi) may be repeated as a unit cycle until a thin film of the desired thickness is obtained. In this way, in one cycle, when the shielding compound of the present invention is injected after the precursor compound and is absorbed into the substrate, the shielding compound may act as a growth activator for thin film formation. In this case, the thin film growth rate may be increased, and the density and crystallinity of the thin film may be increased, thereby reducing the resistivity of the thin film and improving the electrical properties of the thin film.

As a preferred example, in the method of forming a thin film according to the present invention, in one cycle, the shielding compound of the present invention may be introduced before the precursor compound and absorbed onto the substrate. In this case, even when depositing a thin film at high temperatures, process by-products may be significantly reduced and step coverage may be significantly improved by appropriately reducing the thin film growth rate. In addition, the crystallinity of the thin film may be increased, thereby reducing the resistivity of the thin film. In addition, even when applied to semiconductor devices with a large aspect ratio, the thickness uniformity of the thin film may be greatly improved, thereby ensuring the reliability of the semiconductor device.

For example, in the method of forming a thin film, when the shielding compound is deposited before or after the deposition of the precursor compound, depending on the needs, the unit cycle may be repeated 1 to 99,999 times, preferably 10 to 10,000 times, more preferably 50 to 5,000 times, still more preferably 100 to 2,000 times. Within this range, the desired thickness of the thin film may be obtained, and the effects intended for the present invention may be sufficiently achieved.

The precursor compound is a compound having Al, Si, Ti, V, Co, Ni, Cu, Zn, Ga, Ge, Se, Zr, Nb, Mo, Ru, Rh, In, Sn, Sb, Te, Hf, Ta, W, Re, Os, Ir, La, Ce, or Nd as a central metal atom and one or more ligands selected from C, N, O, and H. For a precursor having a vapor pressure of 1 mTorr to 100 Torr at 25 °C, the effect of forming a shielded area by the above-described shielding compound may be maximized.

Any compound known in the art may be used as the precursor compound without any particular limitation. For example, a compound containing a cyclopentadienyl group may be used as the precursor compound. Specifically, examples of the hafnium precursor compound may include tris(dimethylamido) cyclopentadienyl hafnium of CpHf(NMe₂)₃ and (methyl-3-cyclopentadienylpropylamino)bis(dimethylamino)hafnium of Cp(CH₂)₃NM₃Hf(NMe₂)₂.

In the present invention, for example, the chamber may be an ALD chamber, a CVD chamber, a PEALD chamber, or a PECVD chamber.

In the present invention, the shielding compound or precursor compound may be vaporized, injected, and then subjected to plasma post-treatment. In this case, the growth rate of thin film may be improved and process by-products may be reduced.

When the shielding compound is first adsorbed on the substrate and then the precursor compound is adsorbed, or when the precursor compound is first adsorbed on the substrate and then the shielding compound is adsorbed, the amount of purge gas injected into the chamber in the step of purging the unadsorbed shielding compound is not particularly limited as long as the amount of purge gas is sufficient to remove the unadsorbed shielding compound. For example, the amount of purge gas may be 10 to 100,000 times, preferably 50 to 50,000 times, more preferably 100 to 10,000 times. Within this range, by effectively removing the unadsorbed shielding compound, a thin film may be formed evenly and deterioration of film quality may be prevented. Here, the input amounts of the purge gas and shielding compound are each based on one cycle, and the volume of the shielding compound refers to the volume of the vaporized shielding compound.

As a specific example, the shielding compound is injected (per cycle) at a flow rate of 1.66 mL/s for an injection time of 0.5 seconds. In the step of purging the unadsorbed shielding compound, when purge gas is injected (per cycle) at a flow rate 166.6 mL/s for an injection time 3 seconds, the injection amount of purge gas is 602 times the injection amount of shielding compound.

In addition, in the step of purging the unadsorbed precursor compound, the amount of purge gas injected into the chamber is not particularly limited as long as the amount is sufficient to remove the unadsorbed precursor compound. For example, the amount may be 10 to 10,000 times, preferably 50 to 50,000 times, more preferably 100 to 10,000 times the volume of the precursor compound injected into the chamber. Within this range, by sufficiently removing the unadsorbed precursor compound, a thin film may be formed evenly and deterioration of film quality may be prevented. Here, the input amounts of the purge gas and precursor compound are each based on one cycle, and the volume of the precursor compound refers to the volume of the vaporized precursor compound vapor.

In addition, in the purging step performed immediately after the reaction gas supply step, the amount of purge gas injected into the ALD chamber may be 10 to 10,000 times, preferably 50 to 50,000 times, more preferably 100 to 10,000 times the volume of reaction gas injected into the ALD chamber. Within this range, the desired effects may be sufficiently achieved. Here, the input amounts of purge gas and reaction gas are based on one cycle.

The shielding compound and the precursor compound may be transferred into the ALD chamber preferably by a VFC, DLI, or LDS method, more preferably by an LDS method.

For example, the substrate loaded into the chamber may be heated to 50 to 400 °C, as a specific example, 50 to 400 °C. The shielding compound or the precursor compound may be injected onto the substrate in an unheated or heated state, or may be injected unheated and then heated during the deposition process, depending on the deposition efficiency. For example, the shielding compound or the precursor compound may be injected onto the substrate at 50 to 400 °C for 1 to 20 seconds.

The ratio of amount (mg/cycle) of the shielding compound and the precursor compound fed into the chamber may be preferably 1:1.5 to 1:20, more preferably 1:2 to 1:15, still more preferably 1:2 to 1:12, still more preferably 1:2.5 to 1:10. Within this range, step coverage may be improved, and process by-products may be greatly reduced.

In the present invention, for example, the precursor compound may be mixed with a non-polar solvent and introduced into the chamber. In this case, the viscosity or vapor pressure of the precursor compound may be easily controlled.

The non-polar solvent may preferably include one or more selected from the group consisting of alkanes and cycloalkanes. In this case, an organic solvent with low reactivity and solubility and easy moisture management may be included. In addition, step coverage may be improved even when deposition temperature increases during thin film formation.

As a more desirable example, the non-polar solvent may include a C1 to C10 alkane or a C3 to C10 cycloalkane, preferably a C3 to C10 cycloalkane. In this case, reactivity and solubility may be low and moisture management may be easy.

In the present disclosure, C1, C3, and the like indicate the number of carbon atoms.

The cycloalkane may preferably include C3 to C10 monocycloalkanes, and among the monocycloalkanes, cyclopentane is liquid at room temperature and has the highest vapor pressure, so cyclopentane is preferable in the vapor deposition process, but the present invention is not limited thereto.

For example, the non-polar solvent may have a solubility (25°C) of 200 mg/L or less, preferably 50 to 400 mg/L, more preferably 135 to 175 mg/L in water. Within this range, the reactivity toward the precursor compound may be reduced, and moisture may be easily managed.

In the present disclosure, solubility may be measured without any particular limitation by a measurement method or standard commonly used in the technical field to which the present invention pertains. For example, solubility may be measured by the HPLC method using a saturated solution.

Based on a total weight of the precursor compound and the non-polar solvent, the non-polar solvent may be included in an amount of 5 to 95 % by weight, more preferably 10 to 90 % by weight, still more preferably 40 to 90 % by weight, most preferably 70 to 90 % by weight.

When the content of the non-polar solvent exceeds the above range, impurities may be generated, which may increase the resistance and impurities within a thin film. When the content of the organic solvent is less than the range, the effect of improving step coverage due to solvent addition and the effect of reducing impurities such as chloride (Cl) ions may be reduced.

For example, in the method of forming a thin film, when the shielding compound, preferably the compounds represented by Chemical Formulas 1 and 2 are used, the deposition rate reduction rate calculated by Equation 1 below may be 30 % or more, as a specific example, 35 % or more, preferably 38 % or more. In this case, by forming a deposition layer with a uniform thickness due to the difference in the adsorption distribution of the shielding agent having the aforementioned structure as a shielded area that does not remain in a thin film, as a relatively coarse thin film is formed, the growth rate of a thin film formed at the same time is greatly reduced, so that even when applied to a substrate having a complex structure, the uniformity of the thin film may be secured, and the step coverage may be greatly improved. In particular, deposition in a thin thickness is possible, and the remaining amounts of O, Si, metals, and metal oxides remaining as process by-products may be improved. In addition, even the remaining amount of carbon, which was difficult to reduce in the past, may be improved. Deposition rate reduction rate = [{ (DRn)-(DRw)}/(DRn)] × 100

In Equation 1, DRn is the depth rate measured on the manufactured thin film without adding the shielding compound, and DRw is the depth rate measured on the manufactured thin film with the addition of the shielding compound. Here, the depth rate is measured at room temperature and pressure using an ellipsometer for a thin film with a thickness of 3 to 30 nm, and the unit is Å/cycle.

In the method of forming a thin film, the residual halogen intensity (c/s) in the thin film may be preferably 100,000 or less, more preferably 70,000 or less, still more preferably 50,000 or less, still more preferably 10,000 or less, as a preferred example, 5,000 or less, still more preferably 1,000 to 4,000, still more preferably 1,000 to 3,800 as measured using a thin film having a thickness of 100 Å according to SIMS. Within this range, corrosion and deterioration may be effectively prevented.

In the present disclosure, purging may be performed at preferably 1,000 to 50,000 sccm (standard cubic centimeter per minute), more preferably 2,000 to 30,000 sccm, still more preferably 2,500 to 15,000 sccm. Within this range, the thin film growth rate per cycle may be appropriately controlled. In addition, since deposition is performed as an atomic mono-layer or nearly an atomic mono-layer, the film quality may be improved.

The atomic layer deposition (ALD) process is very advantageous in manufacturing integrated circuits (ICs) that require a high aspect ratio. In particular, the ALD process has advantages such as excellent conformality, uniformity, and precise thickness control due to the self-limiting thin film growth mechanism.

For example, the method of forming a thin film may be performed at a deposition temperature of 50 to 800 °C, preferably 300 to 700 °C, more preferably 400 to 650 °C, still more preferably 400 to 600 °C, still more preferably 450 to 600 °C. Within this range, a thin film with excellent film quality may be grown while implementing ALD process characteristics.

For example, the method of forming a thin film may be performed at a deposition pressure of 0.01 to 20 Torr, preferably 0.1 to 20 Torr, more preferably 0.1 to 10 Torr, most preferably 1 to 7 Torr. Within this range, a thin film having a uniform thickness may be obtained.

In the present disclosure, the deposition temperature and the deposition pressure may be measured as temperature and pressure formed within the deposition chamber, or as temperature and pressure applied to the substrate within the deposition chamber.

The method of forming a thin film may preferably include a step of increasing the temperature inside the chamber to the deposition temperature before introducing the shielding compound into the chamber; and/or a step of purging by injecting an inert gas into the chamber before introducing the shielding compound into the chamber.

In addition, as a thin film manufacturing device capable of implementing the thin film manufacturing method, the present invention may include a thin film manufacturing device including an ALD chamber, a first vaporizer for vaporizing a shielding compound, a first transport means for transporting the vaporized shielding compound into the ALD chamber, a second vaporizer for vaporizing a thin film precursor, and a second transport means for transporting the vaporized thin film precursor into the ALD chamber. Here, vaporizers and transport means commonly used in the technical field to which the present invention pertains may be used in the present invention without particular limitation.

As a specific example, the method of forming a thin film is explained. First, a substrate on which a thin film is to be formed is placed in a deposition chamber capable of atomic layer deposition.

The substrate may include a semiconductor substrate, such as a silicon substrate or a silicon oxide substrate.

The substrate may further have a conductive layer or an insulating layer formed on the upper portion thereof.

To deposit a thin film on the substrate positioned in the deposition chamber, the above-described shielding compound, a precursor compound, or a mixture of the precursor compound and a non-polar solvent is prepared.

Then, the prepared shielding compound is injected into a vaporizer, is transformed into a vapor phase, is transferred into a deposition chamber, and is adsorbed on a substrate. Then, purging is performed to remove the unadsorbed shielding compound.

Next, the prepared precursor compound or mixture of the precursor compound and a non-polar solvent (composition for forming a thin film) is injected into the vaporizer, is transformed into a vapor phase, is transferred into the deposition chamber, and is adsorbed on the substrate. Then, the unadsorbed precursor compound/composition for forming a thin film is purged.

In the present disclosure, when necessary, the process of removing the unadsorbed shielding compound by purging after adsorbing the shielding compound on the substrate; and the process of adsorbing the precursor compound onto the substrate and purging to remove the unadsorbed precursor compound may be performed in reverse order.

In the present disclosure, for example, the shielding compound and the precursor compound (composition for forming a thin film) may be delivered into the deposition chamber by vapor flow control (VFC) using mass flow control (MFC) or a liquid delivery system (LDS) using liquid mass flow control (LMFC), preferably an LDS method.

At this time, a mixed gas of one or more selected from the group consisting of argon (Ar), nitrogen (N₂), and helium (He) may be used as a carrier gas or dilution gas to move the shielding compound and the precursor compound onto the substrate, without being limited thereto.

In the present disclosure, for example, an inert gas, preferably the carrier gas or dilution gas may be used as the purge gas.

Next, a reaction gas is supplied. A reaction gas commonly used in the technical field to which the present invention pertains may be used in the present invention without particular limitation. Preferably, the reaction gas may contain a nitriding agent. The nitriding agent and the precursor compound adsorbed on the substrate react to form a nitride film.

Preferably, the nitriding agent may be nitrogen gas (N₂), hydrazine gas (N₂H₄), or a mixture of nitrogen gas and hydrogen gas.

Next, unreacted residual reaction gas is purged using an inert gas. Accordingly, in addition to excess reaction gas, generated byproducts may also be removed.

As described above, in the method of forming a thin film, for example, a step of shielding using a shielding compound on a substrate, a step of purging the unadsorbed shielding compound, a step of adsorbing a precursor compound/composition for forming a thin film on the substrate, a step of purging the unadsorbed precursor compound/composition for forming a thin film, a step of supplying a reaction gas, and a step of purging the residual reaction gas may be set as a unit cycle. To form a thin film of desired thickness, the unit cycle may be repeated.

As another example, in the method of forming a thin film, a step of adsorbing a precursor compound/composition for forming a thin film on the substrate, a step of purging the unadsorbed precursor compound/composition for forming a thin film, a step of adsorbing a shielding compound on the substrate, a step of purging the unadsorbed shielding compound, a step of supplying a reaction gas, and a step of purging the residual reaction gas may be set as a unit cycle. To form a thin film of desired thickness, the unit cycle may be repeated.

For example, the unit cycle may be repeated 1 to 99,999 times, preferably 10 to 1,000 times, more preferably 50 to 5,000 times, still more preferably 100 to 2,000 times. Within this range, the desired thin film properties may be well expressed.

In addition, the present invention provides a semiconductor substrate, and the semiconductor substrate is fabricated by the thin film formation method. In this case, the step coverage and thickness uniformity of a thin film may be excellent, and density and electrical properties may be excellent.

The manufactured thin film may preferably have a thickness of 20 nm or less, a resistivity value of 50 to 400 µΩ·cm based on a thin film thickness of 10 nm, a halogen content of 10,000 ppm or less, and a step coverage of 90 % or more. Within this range, the thin film has excellent performance as a diffusion barrier and has the effect of reducing corrosion of metal wiring materials, without being limited thereto.

For example, the thin film may have a thickness of 0.1 to 20 nm, preferably 1 to 20 nm, more preferably 3 to 25 nm, still more preferably 5 to 20 nm. Within this range, thin film properties may be excellent.

For example, based on a thin film thickness of 10 nm, the thin film may have a resistivity value of 0.1 to 400 µΩ·cm, preferably 15 to 300 µΩ·cm, more preferably 20 to 290 µΩ·cm, still more preferably 25 to 280 µΩ·cm. Within this range, thin film properties may be excellent.

The thin film may have a halogen content of preferably 10,000 ppm or less or 1 to 9,000 ppm, still more preferably 5 to 8,500 ppm, still more preferably 100 to 1,000 ppm. Within this range, thin film properties may be excellent, and thin film growth rate may be reduced. Here, the halogen remaining in the thin film may be, for example, Cl₂, Cl, or Cl⁻. As the halogen residue within the thin film decreases, the film quality increases.

For example, the thin film may have a step coverage of 90 % or more, preferably 92 % or more, more preferably 95 % or more. Within this range, since even a thin film of complex structure may be easily deposited on a substrate, the thin film may be applied to next-generation semiconductor devices.

For example, when necessary, the thin film may have a multilayer structure of two or three layers. As a specific example, the multilayer film with a two-layer structure may have a lower layer-middle layer structure, and the multilayer film with a three-layer structure may have a lower layer-middle layer-upper layer structure.

For example, the lower layer may be formed of one or more selected from the group consisting of Si, SiO₂, MgO, Al₂O₃, CaO, ZrSiO₄, ZrO₂, HfSiO₄, Y₂O₃, HfO₂, LaLuO₂, Si₃N₄, SrO, La₂O₃, Ta₂O₅, BaO, and TiO₂.

For example, the middle layer may be formed of TiₓN_{y}, preferably TN.

For example, the upper layer may be formed of one or more selected from the group consisting of W and Mo.

Hereinafter, preferred examples and drawings are presented to help understand the present invention, but the following examples and drawings are only illustrative of the present invention, and it is obvious to those skilled in the art that various changes and modifications are possible within the scope and technical idea of the present invention. Such changes and modifications fall within the scope of the appended patent claims.

### [Examples]

### Examples 1 to 8, Comparative Examples 1 to 4

An ALD deposition process was performed using the components shown in Table 1 below.

Specifically, as the shielding compound, compounds represented by Chemical Formulas 1-1 to 1-4 below, compounds represented by Chemical Formulas 2-1 to 2-4 below, and compounds represented by Chemical Formulas 3-1 and 3-2 below were prepared.

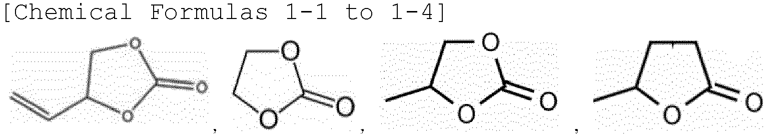

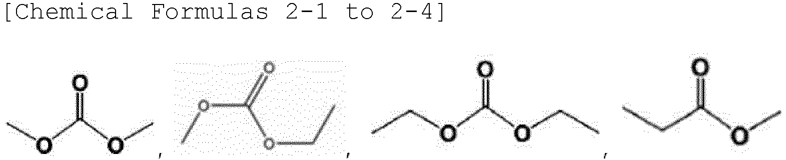

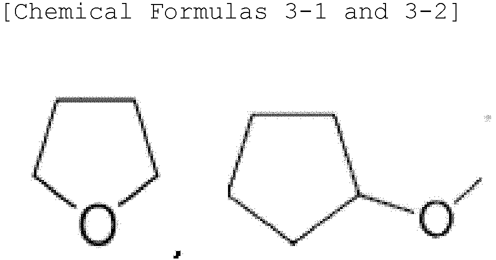

In addition, as the precursor, tris(dimethylamino)cyclopentadienyl hafnium (CpHf(NMe₂)₃, indicated as CpHf in the table below) and (methyl-3-cyclopentadienylpropylamino)bis(dimethylamino)hafnium of Cp(CH₂)₃NM₃Hf(NMe₂)₂ (indicated as H03 in the table below), and TiCl₄ were prepared.

The prepared shielding compound was placed in a canister and supplied to a vaporizer heated to 150 °C at a flow rate of 0.05 g/min using a liquid mass flow controller (LMFC) at room temperature. The shielding compound vaporized from the vaporizer was injected into a deposition chamber loaded with a substrate for 1 second, and then argon gas was supplied at 5000 sccm for 2 seconds to perform argon purging. At this time, the pressure inside the reaction chamber was controlled to 2.5 Torr. Next, the prepared precursor compound was placed in a separate canister and supplied to a separate vaporizer heated to 150 °C at a flow rate of 0.05 g/min using a liquid mass flow controller (LMFC) at room temperature. After Si₂Cl₆ vaporized from the vaporizer was injected into the deposition chamber for 1 second, argon gas was supplied at 5000 sccm for 2 seconds to perform argon purging. At this time, the pressure inside the reaction chamber was controlled to 2.5 Torr.

Next, ammonia or ozone as a reactive gas was injected into the reaction chamber at 1000 sccm for 3 seconds, followed by argon purging for 3 seconds. At this time, the substrate on which a metal thin film is to be formed was heated under the temperature conditions shown in Table 1 below.

By repeating this process 200 to 400 times, a self-limiting atomic layer thin film with a thickness of 10 nm was formed.

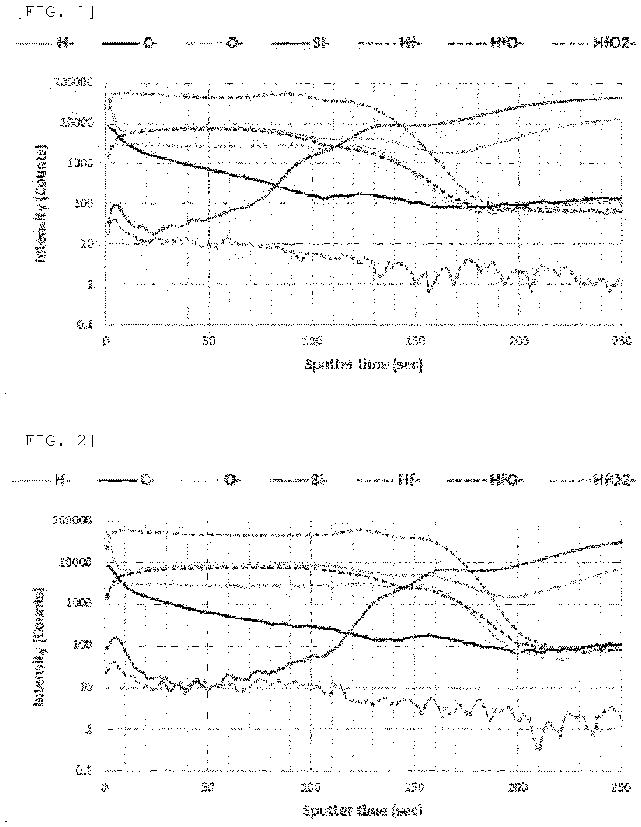

For each thin film obtained in Examples 1 to 8 and Comparative Examples 1 to 4, deposition rate reduction rate (D/R reduction rate) and SIMS C impurities were measured in the following methods, and the results are shown in Table 1 and FIGS. 1 and 2 below.

*Deposition rate reduction rate (D/R (dep. rate) reduction rate): The deposition rate reduction rate represents the rate of reduction in the deposition rate after the shielding agent is added compared to the deposition rate before the shielding agent is added, and was calculated as a percentage using the measured A/cycle values.

Specifically, using an ellipsometer which is a device capable of measuring optical properties such as thickness and refractive index of the thin film by using the polarization characteristics of light for the manufactured thin film, the thickness of the thin film was measured. Then, the thickness of the thin film deposited per cycle was calculated by dividing the measured thickness by the number of cycles. Based on these results, the thin film growth rate reduction rate was calculated. Specifically, the calculation was performed using Equation 1 below. Deposition rate reduction rate = [{(DRn)-(DRw)}/(DRn)] × 100

In Equation 1, DRn is the depth rate measured on the manufactured thin film without adding the shielding compound, and DRw is the depth rate measured on the manufactured thin film with the addition of the shielding compound. Here, the depth rate is measured at room temperature and pressure using an ellipsometer for a thin film with a thickness of 3 to 30 nm, and the unit is Å/cycle.
*SIMS (Secondary-ion mass spectrometry) C impurities: The C impurity value was checked from the SIMS graph by considering the C impurity content (counts) when the thin film was axially penetrated by an ion sputter and the sputter time was 50 seconds with little contamination on the substrate surface layer.

**[Table 1]**

| Classific ation | Film quality classific ation | Shield ing agent (Chemi cal Formul a No.) | Shield ing agent carrie r (sccm) | Precur sor types | React ant types | Deposit ion tempera ture (°C) | D/R reduct ion rate (%) | SIMS C impurit ies (counts ) |
|---|---|---|---|---|---|---|---|---|
| Comparati ve Example 1 | HfO₂ | Equati on 3-1 | 50 | CpHf | O₃ | 320 | 8.0 | 21,369 |
| Comparati ve Example 2 | HfO₂ | Equati on 3-2 | 50 | CpHf | O₃ | 320 | 9.3 | - |
| Comparati ve Example 3 | HfO₂ | Equati on 3-2 | 50 | CpHf | O₃ | 360 | 10.9 | - |
| Comparati ve Example 4 | HfO₂ | - | - | H03 | O₃ | 340 | 0 | 630 |
| Example 1 | HfO₂ | Equati on 2-2 | 10 | CpHf | O₃ | 340 | 45.0 | - |
| Example 2 | HfO₂ | Equati on 2-1 | 10 | H03 | O₃ | 340 | 44.9 | - |
| Example 3 | HfO₂ | Equati on 2-1 | 10 | H03 | O₃ | 380 | 49.6 | - |
| Example 4 | HfO₂ | Equati on 2-1 | 50 | H03 | O₃ | 380 | 88.5 | 662 |
| Example 5 | HfO₂ | Equati on 1-3 | 10 | H03 | O₃ | 400 | 50.8 | - |
| Example 6 | HfO₂ | Equati on 1-3 | 10 | H03 | O₃ | 440 | 48.2 | - |
| Example 7 | TiN | Equati on 2-3 | 10 | TiCl₄ | NH₃ | 460 | 39.4 | - |
| Example 8 | HfO₂ | Equati on 2-1 | 50 | H03 | O₃ | 360 | 85.2 | - |

As shown in Table 1 and FIGS. 1 and 2 below, compared to Comparative Example 4 without using the shielding compound of the present invention, in Example 4 using the shielding compound of the present invention, the deposition rate reduction rate was significantly improved, and impurity reduction characteristics was excellent. In addition, compared to Comparative Examples 1 to 3 using compounds different from the shielding compound of the present invention, such as THF and CPME, in Examples 1 to 3 and Examples 5 to 8 using the shielding compound of the present invention, the deposition rate reduction rate was significantly improved, and impurity reduction characteristics was excellent.

In particular, compared to Comparative Example 4 without using the shielding compound of the present invention or Comparative Examples 1 to 3 using THF and CPME, in Examples 1 to 8 using the shielding compound of the present invention, the reduction rate in thin film growth rate per cycle (20 % or more) was excellent.

### Additional Experimental Examples

### Determination of internal oxidation of thin film

To determine the internal states of the thin film manufactured in Example 8 and the thin film manufactured in Comparative Example 3, the hafnium (Hf) to oxygen (O) ratio was analyzed.

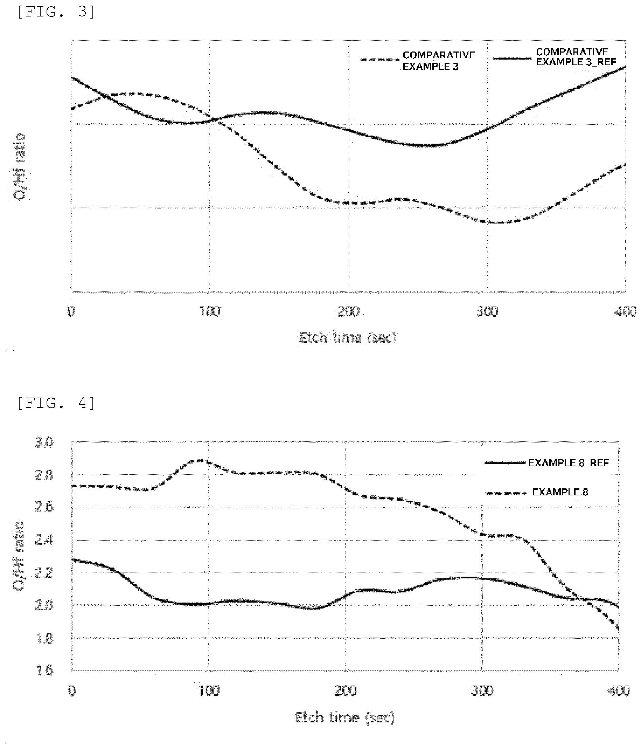

Specifically, for the thin films with a thickness of about 10 nm manufactured in Example 8 and Comparative Example 3, Hf and O elements were analyzed by X-ray photoelectron spectroscopy (XPS) while digging in the depth direction, and the elemental amounts (at %) of Hf and O elements were converted into a ratio (O/Hf ratio). The results are shown in FIGS. 3 and 4 below.

FIG. 3 below is a graph of the hafnium (Hf) to oxygen (O) ratio analysis of the thin film manufactured in Comparative Example 3, and FIG. 4 below is a graph of the hafnium (Hf) to oxygen (O) ratio analysis of the thin film manufactured in Example 8. As the etch time increases, it indicates the oxidation state deep inside the HfO₂ thin film.

As shown in FIG. 4, when the shielding compound of Chemical Formula 2-1 is significantly reduced to reach a deposition rate of 88.5 %, the number of exposures to reactant O₃ increases by 2 to 4 times compared to the conventional method, which indicates a sufficient oxidation number.

For reference, when Hf exists in a metallic state, the dielectric constant, which is very important as a dielectric, is reduced. Considering that the stoichiometric ratio of HfO₂ is hafnium (1): oxygen (2), and the simulation result of the O/Hf ratio before applying the shielding agent is 1.9, looking at the midpoint (etch time @150 sec) of 5 nm thickness in the depth direction of a 10 nm thin film to which the Chemical Formula 2-1 compound of Example 8 was applied as a shielding agent, which is free from external influence, it was analyzed that the O/Hf ratio was 2.0, with 10% more oxidation compared to before applying the shielding agent.

On the other hand, as shown in FIG. 3 below, when the deposition rate (D/R) is reduced by using the shielding compound of Chemical Formula 3-2, it can be confirmed that Hf is less oxidized compared to the conventional process without applying the shielding agent. Considering the stoichiometric ratio of HfO₂ described above and the simulated value of O/Hf ratio of 1.9 before applying the shielding agent, looking at the midpoint (etch time @200 sec) of 5 nm thickness in the depth direction of a 10 nm thin film to which the Chemical Formula 3-2 compound of Comparative Example 3 was applied as a shielding agent, which is free from external influence, the oxidation number was analyzed to be 3.75 % less than before applying the CPME shielding agent, and the O/Hf ratio was analyzed to be 1.83.

That is, when the dotted line is relatively higher than the solid line, it can be considered close to HfO2.0, and the higher it is, the better it can be interpreted. It can be seen that the internal oxidation degree of the thin film is effectively improved when the shielding compound of the present invention is applied.

### Evaluation of step coverage and thickness uniformity

The step coverage was confirmed in the thin film manufactured in Example 4 and the thin film manufactured in Example 5, respectively.

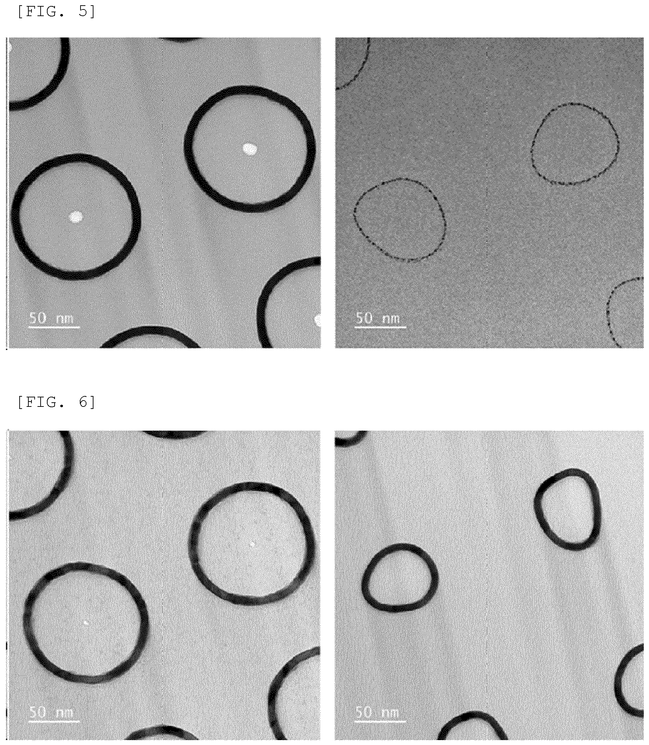

FIG. 5 below shows TEM measurements of a specimen cut horizontally at a position 100 nm downward from the upper portion (left drawing) and at a position 100 nm upward from the lower portion (right drawing) of a thin film deposited under the conditions of Example 4 on a substrate of a complex structure with an aspect ratio of 22:1.

Specifically, on a substrate of a complex structure with an upper portion diameter of 90 nm, a lower portion diameter of 65 nm, a via hole depth of approximately 2000 nm, an aspect ratio of 22:1, the deposition process was performed using the shielding agent application conditions of Example 4.

To confirm the thickness uniformity and step coverage deposited inside a vertically formed via hole, a specimen was prepared by cutting horizontally at a position 100 nm downward from the upper portion and at a position 100 nm upward from the lower portion, and was analyzed using a transmission electron microscope (TEM).

Here, a drawing of a position measurement 100 nm downward from the upper portion is shown as the left drawing of FIG. 5, and a drawing of a position measurement 100 nm upward from the lower portion is shown as the right drawing of FIG. 5.

In FIG. 5 below, the light background represents SiO₂, and the dark black represents HfO₂. Considering that the thickness deposited on the inner wall of the upper portion of the via hole was approximately 8 nm and the thickness deposited on the inner wall of the lower portion was approximately 1.5 nm, the step coverage was analyzed to be 18.75 %.

In particular, as shown in the right drawing, it can be seen that the black lines lack continuity, which confirms the absence of uniformity in the narrow and deep complex structure.

FIG. 6 below is a TEM measurement diagram of a specimen cut horizontally at a position 100 nm downward from the upper portion (left diagram) and at a position 100 nm upward from the lower portion (right diagram) of a thin film deposited under the shielding compound application conditions of Chemical Formula 1-3 of Example 5 on a substrate of a complex structure with an aspect ratio of 22:1.

Specifically, on a substrate of a complex structure with an upper portion diameter of 90 nm, a lower portion diameter of 65 nm, a via hole depth of approximately 2000 nm, an aspect ratio of 22:1, the deposition process was performed using the shielding agent application conditions of Example 4.

To confirm the thickness uniformity and step coverage deposited inside the vertically formed via hole, a specimen was prepared by cutting horizontally at a position 100 nm downward from the upper portion and at a position 100 nm upward from the lower portion, and was analyzed using a transmission electron microscope (TEM).

Here, a drawing of a position measurement 100 nm downward from the upper portion is shown as the left drawing of FIG. 6, and a drawing of a position measurement 100 nm upward from the lower portion is shown as the right drawing of FIG. 6.

In FIG. 6 below, the light background represents SiO₂, and the dark black represents HfO₂. Considering that the thickness deposited on the inner wall of the upper portion of the via hole was approximately 7.7 nm and the thickness deposited on the inner wall of the lower portion was approximately 7.6 nm, the step coverage was analyzed to be 98.7 %.

In particular, the continuity of the black lines in both the left and right drawings is clear, indicating excellent uniformity in narrow and deep complex structures.

As described above, after examining the step coverage and thickness uniformity, it was confirmed that, in Example 4, which used the shielding compound of Chemical Formula 2-1, and Example 5, which used the shielding compound of Chemical Formula 1-3, Example 5 provided better step coverage and thickness uniformity than Example 4.

### Thin film crystallinity

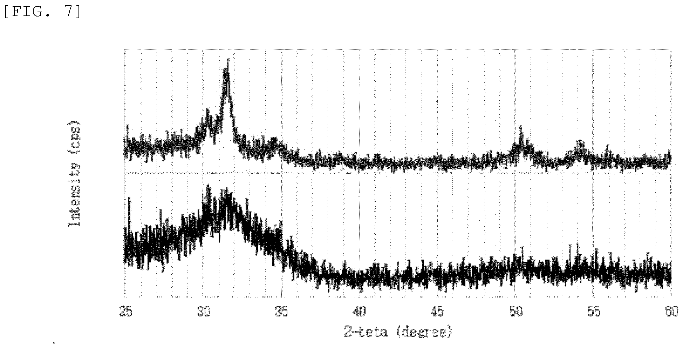

XRD analysis was performed for the case where no shielding compound was added in Comparative Example 4 and the case where the shielding compound was added at an amount of 0.1 g/min before the thin film precursor compound was added in Example 1, and the results are shown in FIG. 7 below.

FIG. 7 below is a diagram showing the XRD crystal patterns of Comparative Example 4 (black) and Example 1 (blue) .

As shown in FIG. 7, in Example 1, when the shielding compound was added at an amount of 0.1 g/min before the thin film precursor compound was added, it was confirmed that the crystal grains of the thin film became larger, i.e., the crystallinity increased.

Here, the grain size may be identified by the width of the peak at 31.5° of the HfO₂ thin film. As the width of the peak decreases, the crystallinity increases. As the crystallinity increases, the dielectric constant may be significantly improved. In addition, the characteristic peak of tetragonal, which has the highest dielectric constant among monoclinic and orthorhombic (tetragonal) systems, was observed at 30.5°.

### Thin film density

X-ray reflectometry (XRR) analysis was performed for the case where no shielding compound was added in Comparative Example 4 and the case where the shielding compound was added at an amount of 0.1 g/min before the thin film precursor compound was added in Example 4, and the results are shown in Table 1 below.

In addition, the same process as Example 4 was repeated except that the deposition temperature was changed to 340 degrees and 420 degrees, and then reflectometry (XRR) analysis was performed. The results are shown in Example 4-1 and Example 4-2.

In addition, the same process as in Comparative Example 4 was repeated except that the deposition temperature was changed to 380 degrees and 420 degrees, and then reflectometry (XRR) analysis was performed. The results are shown in Comparative Example 4-1 and Comparative Example 4-2.

**[Table 2]**

| Experimental Examples | Deposition temperature (°C) | Density (g/cm³) |
|---|---|---|
| Comparative Example 4 | 340 | 10.34 |
| Comparative Example 4-1 | 380 | 10.48 |
| Comparative Example 4-2 | 420 | 10.47 |
| Example 4-1 | 340 | 11.00 |
| Example 4 | 380 | 10.95 |
| Example 4-2 | 420 | 11.15 |

As shown in Table 2, in Example 4, Example 4-1, and Example 4-2, when shielding was performed by adding the shielding compound at a rate of 0.1 g/min before adding the thin film precursor compound, compared to Comparative Example 4, Comparative Example 4-1, and Comparative Example 4-2, thin film density was greatly improved.

In conclusion, the present invention may provide a shielding compound, a method of forming a thin film using the shielding compound, a semiconductor substrate fabricated using the method, and a semiconductor device including the semiconductor substrate. According to the present invention, by providing a compound having a predetermined structure as a shielding agent, by forming a deposition layer with a uniform thickness as a shielded area on a substrate due to the difference in adsorption distribution of the shielding agent, the deposition rate of the thin film may be reduced, and the thin film growth rate may be appropriately reduced. In addition, even when forming a thin film on a substrate with a complex structure, step coverage and the thickness uniformity of a thin film may be improved, and impurities may be greatly reduced.

## Claims

1. A shielding compound having two or more types of nitrogen (N), oxygen (O), phosphorus (P), and sulfur (S) and comprising a linear or cyclic saturated or unsaturated hydrocarbon having 3 to 15 carbon atoms.

2. The shielding compound according to claim 1, wherein the shielding compound has a structure containing nitrogen (N), oxygen (O), phosphorus (P), or sulfur (S) at each terminal of a central carbon atom connected by a double bond to oxygen.

3. The shielding compound according to claim 1, wherein the thin film shielding compound has a structure containing nitrogen (N), oxygen (O), phosphorus (P), or sulfur (S) at one end of a central carbon atom connected by a double bond to oxygen and carbon (C) at the other end.

4. The shielding compound according to claim 1, wherein the shielding compound comprises one or more selected from a compound represented by Chemical Formula 1 below and a compound represented by Chemical Formula 2 below. 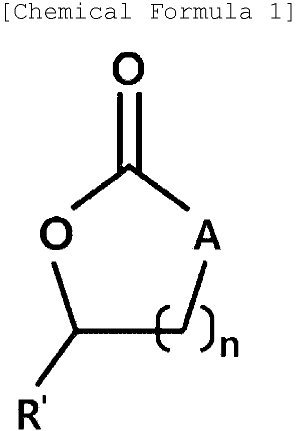 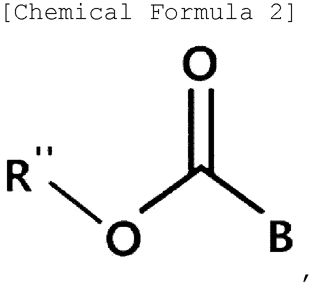
wherein A is oxygen (O), sulfur (S), phosphorus (P), nitrogen (N), -CH, or -CH₂;
B is -OH, -OCH₃, -OCH₂CH₃, -CH₂CH₃, -SH, -SCH₃, or - SCH₂CH₃;
R' and R'' are independently hydrogen, an alkyl group having 1 to 5 carbon atoms, an alkene group having 1 to 5 carbon atoms, or an alkoxy group having 1 to 5 carbon atoms; and
n is an integer from 0 to 3.

5. The shielding compound according to claim 1, wherein the shielding compound comprises one or more selected from compounds represented by Chemical Formulas 1-1 to 1-4 and Chemical Formulas 2-1 to 2-4 below.

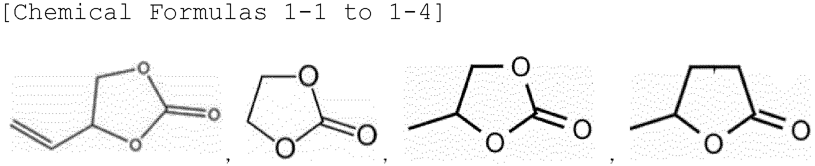

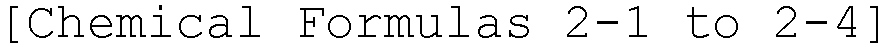

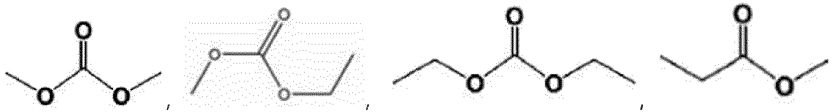

6. The shielding compound according to claim 1, wherein the shielding compound provides a shielded area for an oxide film, a nitride film, a metal film, or a selective thin film thereof, and the shielded area is formed on an entire or part of a substrate on which the oxide film, the nitride film, the metal film, or the selective thin film thereof is formed.

7. The shielding compound according to claim 6, wherein, based on 100 % of a total area of the substrate, the shielded area occupies 10 to 95 % of the entire substrate or a portion of the substrate, and an unshielded area occupies a remainder.

8. The shielding compound according to claim 1, wherein the thin film is a laminated film of one or more selected from the group consisting of Al, Si, Ti, V, Co, Ni, Cu, Zn, Ga, Ge, Se, Zr, Nb, Mo, Ru, Rh, In, Sn, Sb, Te, Hf, Ta, W, Re, Os, Ir, La, Ce, and Nd.

9. The shielding compound according to claim 1, wherein the thin film is used as a diffusion barrier film, an etching stop film, an electrode film, a dielectric film, a gate insulating film, a block oxide film, or a charge trap.

10. A method of forming a thin film, comprising injecting a shielding compound having a structure represented by Chemical Formula 1 or 2 below into a chamber to shield a surface of a loaded substrate. 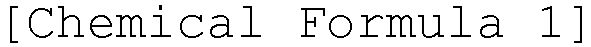 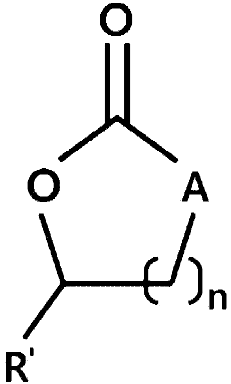 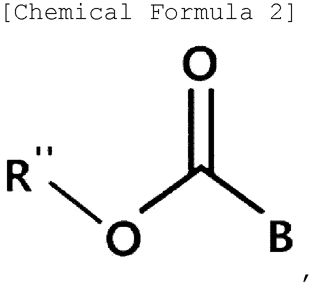
wherein A is oxygen (O), sulfur (S), phosphorus (P), nitrogen (N), -CH, or -CH₂;
B is -OH, -OCH₃, -OCH₂CH₃, -CH₂CH₃, -SH, -SCH₃, or - SCH₂CH₃;
R' and R'' are independently hydrogen, an alkyl group having 1 to 5 carbon atoms, an alkene group having 1 to 5 carbon atoms, or an alkoxy group having 1 to 5 carbon atoms; and
n is an integer from 0 to 3.

11. The method according to claim 10, wherein the chamber is an ALD chamber, a CVD chamber, a PEALD chamber, or a PECVD chamber.

12. The method according to claim 10, wherein the shielding compound is transferred into the chamber by a VFC, DLI, or LDS method, and the thin film is a silicon nitride film, a silicon oxide film, a titanium nitride film, a titanium oxide film, a tungsten nitride film, a molybdenum nitride film, a hafnium oxide film, a zirconium oxide film, a tungsten oxide film, or an aluminum oxide film.

13. A semiconductor substrate fabricated using the method according to claim 10.

14. The semiconductor substrate according to claim 13, wherein the thin film has a multilayer structure of 2 or 3 layers.

15. A semiconductor device comprising the semiconductor substrate according to claim 14.
